# EUROPÄISCHE PATENTSCHRIFT

(11) **EP 0 773 007 B1**
(45) Veröffentlichungstag und Bekanntmachung des Hinweises auf die Patenterteilung: **02.02.2000**
(21) Anmeldenummer: 95117539.7
(22) Anmeldetag: 07.11.1995
(51) Int. Cl.: A61F 2/34

(54) **Endoprothetische Hüftgelenkpfanne**
Endoprosthetic acetabular cup
Capule de hanche endoprothétique

(43) Veröffentlichungstag der Anmeldung: 14.05.1997
(73) Patentinhaber: Aesculap AG & Co. KG, 78532 Tuttlingen (DE)
(72) Erfinder: Blömer, Wilhelm, D 78532 Tuttlingen (DE); Reu, Gerhard, D 78573 Wurmlingen (DE)
(74) Vertreter: Hoeger, Stellrecht & Partner

(56) Entgegenhaltungen:
- EP-A- 0 277 511
- EP-A- 0 457 222
- EP-A- 0 482 320
- WO-A-95/22944
- DE-U- 9 103 574
- DE-U- 9 313 233

## Beschreibung

Die Erfindung betrifft eine endoprothetische Hüftgelenkpfanne mit einer einen nach unten offenen Innenraum aufweisenden Außenschale und einem in diesen einsetzbaren Gelenkeinsatz, der ein kugelige Gelenkfläche aufweist, wobei in den Innenraum der Außenschale ein Ring eingesetzt ist, der in der Außenschale fixiert ist und dessen Innenfläche eine Anlagefläche für den Gelenkeinsatz bildet, der an dem Ring festgelegt ist.

Bei endoprothetischen Hüftgelenkpfannen dieser Art können unterschiedliche Gelenkeinsätze mit der Außenschale kombiniert werden, so daß die Abmessungen und/oder die Positionierung der Gelenkfläche relativ zur Außenschale den Notwendigkeiten angepaßt werden können. Zu diesem Zweck ist es bekannt, unterschiedliche Gelenkeinsätze vorzusehen, deren Gelenkflächen verschieden dimensioniert und/oder verschieden orientiert angeordnet sind, so daß der Operateur durch Auswahl eines bestimmten Gelenkeinsatzes die Größe und Lage der Gelenkfläche relativ zur Außenschale frei wählen kann (EP 091 315 B1). Notwendig ist dabei aber, daß eine größere Anzahl unterschiedlich ausgebildeter Gelenkeinsätze vorgesehen werden, und diese Gelenkeinsätze sind hochwertige und daher teure Teile, so daß ein erheblicher Lagerhaltungsaufwand entsteht.

Aus der EP 0 482 320 A1 ist eine Hüftgelenkpfanne bekannt, bei der in eine Außenschale ein Zwischenring eingesetzt wird, dieser Zwischenring wird mit der Außenschale verbunden und dient der Ausbildung eines Ringraums, in den Knochensubstanz eingefüllt werden kann. Der Gelenkeinsatz wird in diesen Zwischenring eingesetzt, das heißt die Außenschale und der Zwischenring bilden praktisch gemeinsam eine den Gelenkeinsatz aufnehmende Außenschale. Eine Variation der Position der halbkugeligen Gelenkfläche gegenüber der Außenschale ist in dieser Druckschrift nicht erwähnt.

In ähnlicher Weise wird bei einer Hüftgelenkpfanne der Einsatz gebildet aus einem Pfannenkörper aus Kunststoff und einem diesen umgebenden Mantel. Zweck dieser Konstruktion ist es, die Verbindung des Kunststoffeinsatzes einerseits und des metallischen Außenteils andererseits nicht dem Arzt zu überlassen, sondern diese Verbindung bereits fabrikseitig auszuführen, damit der Arzt dann nur noch die Verbindung zwischen dem metallischen Mantel einerseits und der Außenpfanne andererseits vornehmen kann (EP-A-2 277 511).

Es ist Aufgabe der Erfindung, eine gattungsgemäße Hüftgelenkpfanne so auszubilden, daß sie mit geringstmöglichem Aufwand an unterschiedliche Abmessungen und/oder Orientierungen angepaßt werden kann.

Diese Aufgabe wird bei einer endoprothetischen Hüftgelenkpfanne der eingangs beschriebenen Art erfindungsgemäß dadurch gelöst, daß die Innenfläche des in die Außenschale eingesetzten Rings bei verschiedenen Ringen relativ zu der Außenschale unterschiedlich angeordnet ist. Je nach Wahl des Rings ist es daher möglich, den Gelenkeinsatz relativ zur Außenschale unterschiedlich zu positionieren, wobei zur Erreichung verschiedener Positionierungen nur notwendig ist, verschiedene Ringe vorrätig zu halten. Diese Ringe sind einfache Bauteile, die ohne großen Aufwand herstellbar sind.

So kann es beispielsweise vorgesehen sein, daß die Innenfläche des in die Außenschale eingesetzten Rings bei verschiedenen Ringen unterschiedlich tief in die Außenschale eintaucht. Bei Verwendung ein- und desselben Gelenkeinsatzes ist es daher möglich, die Gelenkfläche in unterschiedlicher Tiefe in der Außenschale festzulegen.

Weiterhin kann vorgesehen sein, daß die Innenfläche des in die Außenschale eingesetzten Rings bei verschiedenen Ringen relativ zu der Außenschale unterschiedlich seitlich versetzt angeordnet ist. Damit kann die Gelenkfläche entweder konzentrisch in der Außenschale angeordnet sein oder exzentrisch, die Größe der Exzentrizität läßt sich durch die Auswahl eines bestimmten Rings in einfacher Weise wählen.

Weiterhin kann vorgesehen sein, daß die Innenfläche des in die Außenschale eingesetzten Rings bei verschiedenen Ringen relativ zu der Außenschale unterschiedlich geneigt angeordnet ist. Dadurch ergibt sich die Möglichkeit je nach Verwendung eines bestimmten Rings die Gelenkfläche ohne Neigung in die Außenschale einzusetzen oder mit einer beliebigen Neigung von beispielsweise 3, 5 oder 10°.

Selbstverständlich können durch geeignete geometrische Ausgestaltung der Ringe die genannten unterschiedlichen Anordnungen auch kombiniert verwendet werden, beispielsweise kann ein Gelenkeinsatz durch Wahl eines entsprechenden Rings so in der Außenschale angeordnet sein, daß die Gelenkfläche in einer bestimmten Tiefe seitlich versetzt und geneigt ist, hier hat der Operateur allein durch die Auswahl des entsprechenden Rings eine große Zahl von Möglichkeiten.

Der untere Rand eines Rings kann in verschiedenen Umfangsbereichen einen unterschiedlichen Abstand von einer Horizontalebene der Außenschale aufweisen, so daß der eingesetzte Gelenkeinsatz eine unterschiedliche Abstützung in dem Ring erfährt.

Bei einer besonders bevorzugten Ausführungsform ist vorgesehen, daß die Innenflächen der Ringe gleich geformt und dimensioniert sind. Damit sind alle Ringe geeignet, einen einzigen, in seiner Ausformung und Dimensionierung entsprechend angepaßten Gelenkeinsatz aufzunehmen, der dann bei Auswahl unterschiedlicher Ringe in der Außenschale unterschiedlich positioniert wird. Es genügt also für diese Hüftgelenkpfanne, eine bestimmte Außenschale und einen bestimmten Gelenkeinsatz zu verwenden, die Anpassung in der Positionierung der Gelenkfläche erfolgt allein durch die Auswahl eines bestimmt geformten Rings.

Bei anderen Ausführungsformen kann vorgesehen sein, daß die Innenfläche des in die Außenschale eingesetzten Rings bei verschiedenen Ringen unterschiedlich geformt und/oder dimensioniert ist. Ein solcher Ring ist dann geeignet, eine Anpassung zwischen einer bestimmten Außenschale und einem bestimmten Gelenkeinsatz vorzunehmen; es ist dadurch möglich, verschiedenartige und verschieden große Gelenkeinsätze mit derselben Außenschale zu verwenden. Selbstverständlich kann auch in diesem Falle vorgesehen sein, daß die an den jeweiligen Gelenkeinsatz angepaßte Innenfläche zusätzlich noch so angeordnet ist, daß der jeweilige Gelenkeinsatz relativ zur Außenschale unterschielich positioniert wird, wie dies oben beschrieben worden ist. Man erhält somit eine größtmögliche Variabilität einer Hüftgelenkpfanne, die allein durch Auswahl eines anderen Rings an Gelenkeinsätze der verschiedensten Ausgestaltung angepaßt werden kann und die es auch ermöglicht, die Gelenkflächen der Gelenkeinsätze jeweils in der gewünschten Weise relativ zur Außenschale zu positionieren.

Bei einer bevorzugten Ausführungsform ist vorgesehen, daß der Ring eine konische Außenfläche aufweist, die an einer konischen Innenfläche des Innenraums der Außenschale anliegt. Weiterhin kann vorgesehen sein, daß die Innenfläche konisch ausgebildet ist und daß der Gelenkeinsatz eine konische Außenfläche trägt, die an der Innenfläche anliegt. Bei einer solchen Ausgestaltung werden die konische Außenfläche und die konische Innenfläche des Rings relativ zueinander unterschiedlich angeordnet und gegebenenfalls auch unterschiedlich dimensionert. Die Herstellung ist dabei außerordentlich einfach, es genügt nämlich, einen normalen Ring mit einer inneren und einer äußeren konischen Fläche zu bilden und die Orientierung und/oder Abmessung dieser beiden konischen Flächen unterschiedlich zu wählen.

Die Erfindung bezieht sich nicht nur auf eine Hüftgelenkpfanne, die jeweils aus einer Außenschale, einem Ring und einem Gelenkeinsatz besteht, sondern auch auf einen Bausatz zur Herstellung einer solchen Hüftgelenkpfanne, der eine Außenschale, einen Gelenkeinsatz und eine größere Anzahl von unterschiedlich geformten und/oder dimensionierten Ringen aufweist, so daß bei geeigneter Kombination eine Gelenkpfanne daraus herstellbar ist, bei der die Gelenkfläche relativ zur Außenschale unterschiedlich angeordnet werden kann.

Die nachfolgende Beschreibung bevorzugter Ausführungsformen der Erfindung dient im Zusammenhang mit der Zeichnung der näheren Erläuterung. Es zeigen:
- Figur 1:: eine Ansicht einer Hüftgelenkpfanne mit einer Außenschale, einem Gelenkeinsatz und einem Ring vor dem Zusammensetzen dieser Teile;
- Figur 2:: eine erste bevorzugte Ausführungsform eines Rings zum konzentrischen, ungeneigten Einsetzen eines Gelenkeinsatzes in die Außenschale;
- Figur 3:: eine Ansicht ähnlich Figur 2 mit einer konischen Innenfläche, die in axialer Richtung gegenüber der konischen Innenfläche des Ausführungsbeispiels der Figur 2 verschoben ist;
- Figur 4:: eine Ansicht ähnlich Figur 2 mit einem Ring, dessen konische Innenfläche gegenüber der konischen Außenfläche geneigt ist;
- Figur 5:: eine Ansicht ähnlich Figur 4 mit einer schwächeren Neigung der konischen Innenfläche gegenüber der konischen Außenfläche;
- Figur 6:: eine Ansicht ähnlich Figur 5, wobei die Bauhöhe des Rings an einer Seite größer ist als an der gegenüberliegenden Seite;
- Figur 7:: eine Ansicht ähnlich Figur 2 mit einer konischen Innenfläche, die gegenüber der konischen Außenfläche seitlich versetzt ist.

Die in der Zeichnung dargestellte Hüftgelenkpfanne umfaßt eine im wesentlichen halbkugelige Außenschale 1 mit einem nach unten offenen Innenraum 2, einen in den Innenraum 2 einsetzbaren Ring 3 sowie einen in den Ring 3 einsetzbaren Gelenkeinsatz 4.

Im dargestellten Ausführungsbeispiel ist die Außenschale 1 halbkugelig ausgebildet, grundsätzlich wäre aber auch eine andere Formgebung möglich, beispielsweise könnte die Außenschale 1 eine konische Außenkontur aufweisen. Diese Außenschale 1 kann weiterhin in der Zeichnung nicht dargestellte Gewinderippen tragen, so daß diese Außenschale 1 in an sich bekannter Weise in eine Ausnehmung des Knochens eingesetzt werden kann, beispielsweise durch Einschrauben.

Der Innenraum 2 wird bei dem dargestellten Ausführungsbeispiel an seiner Unterseite begrenzt durch eine konische oder kegelstumpfförmige Innenwand 5, an die sich nach oben hin eine weitere konische oder kegelstumpfförmige, stärker konvergierende Ringwand 6 anschließt. An der Oberseite ist der Innenraum 2 durch eine horizontale Abschlußwand 7 begrenzt.

Im dargestellten Ausführungsbeispiel ist die Außenschale 1 geschlossen, grundsätzlich wäre es aber ohne weiteres möglich, auch eine ringförmige Außenschale 1 zu verwenden, die also an der Oberseite offen ist.

Der Ring 3 weist eine konische oder kegelstumpfförmige Außenfläche 8 auf, deren Konizität der der Innenwand 5 des Innenraums 2 entspricht und die so dimensioniert ist, daß der Ring 3 in den Innenraum 2 eingesetzt werden kann, wobei die Außenfläche 8 flächig an der Innenwand 5 des Innenraums 2 anliegt.

Auf der Innenseite weist der Ring 3 eine ebenfalls konische Innenfläche 9 auf, die eine Anlagefläche für den Gelenkeinsatz 4 bildet.

Dieser Gelenkeinsatz 4, der beispielsweise aus Hochdruckpolyethylen bestehen kann, ist schalenförmig aufgebaut und umgibt eine im wesentlichen halbkugelige, nach unten offene Gelenkfläche 10. An seiner Außenseite wird er begrenzt durch eine konische oder kegelstumpfförmige Außenfläche 11, die sich vom unteren Rand 12 über einen erheblichen Teil der Höhe des Gelenkeinsatzes 4 erstreckt und deren Konizität der der Innenfläche 9 des Rings 3 entspricht. Es ist daher möglich, den Gelenkeinsatz 4 in den Ring 3 einzusetzen, wobei die Außenfläche 11 flächig an der Innenfläche 9 des Gelenkeinsatzes 4 zur Anlage kommt.

Während bei dem in der Zeichnung dargestellten Ausführungsbeispiel nur eine Außenschale 1 und ein Gelenkeinsatz 4 zur Anwendung kommen, können unterschiedlich dimensionierte Ringe 3 verwendet werden, um die Position des Gelenkeinsatzes 4 relativ zur Außenschale 1 unterschiedlich zu gestalten.

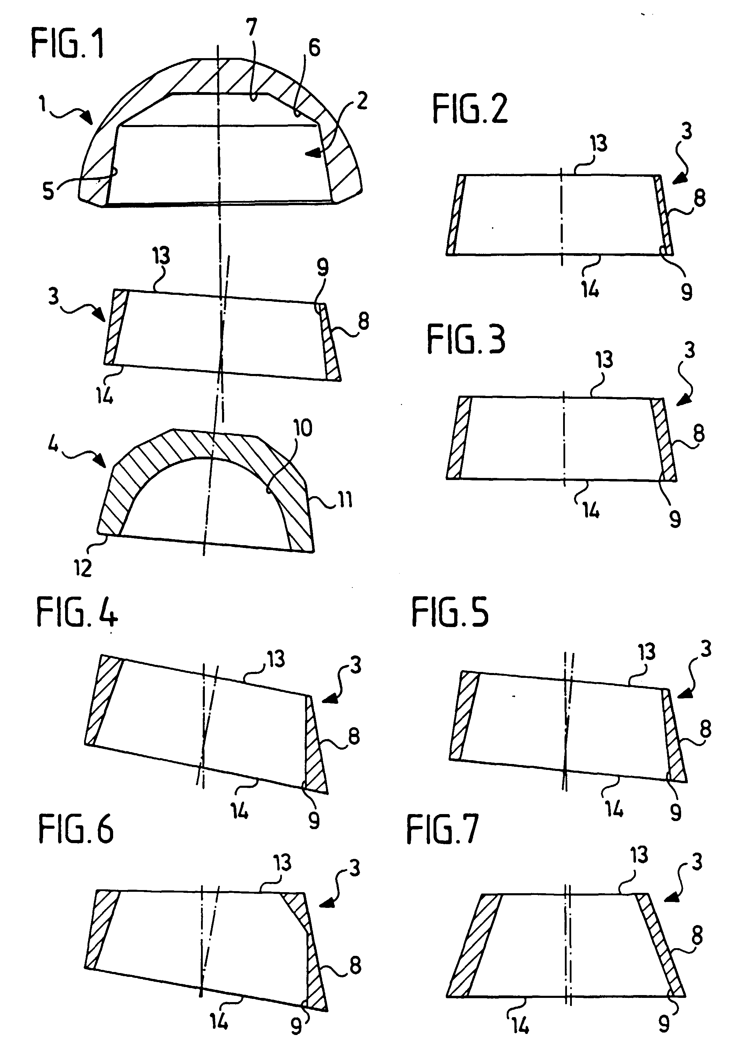

Bei einem ersten Typ eines Rings 3, wie er in den Figuren 2 und 3 dargestellt ist, sind die Außenfläche 8 und die Innenfläche 9 konzentrisch zueinander angeordnet, so daß bei einem in die Außenschale 1 eingesetzten Ring 3 der Gelenkeinsatz 4 koaxial zur Außenschale 1 angeordnet wird.

Bei einem in dieser Weise ausgebildeten Ring 3 kann die Innenfläche 9 gegenüber der Außenfläche 8 in axialer Richtung in unterschiedlicher Weise positioniert sein, so daß ein- und derselbe Gelenkeinsatz 4 bei Verwendung von Ringen dieser unterschiedlichen Dimensionierung unterschiedlich tief in eine Außenschale 1 eingesetzt werden kann. Dies wird beim Vergleich der Ringe der Figuren 2 und 3 deutlich. Beim Ausführungsbeispiel der Figur 3 ist die Innenfläche 9 gegenüber der Außenfläche 8 nach unten verschoben, die Wandstärke wird dadurch größer, und beim Einsetzen des Gelenkeinsatzes 4 kann dieser in dem Ring der Figur 3 nicht so weit nach oben in Richtung auf die Außenschale 1 verschoben werden wie im Falle des Rings der Figur 2. Damit wird es möglich, die Gelenkfläche 10 relativ zur Außenschale 1 in unterschiedlicher Höhe zu positionieren.

Bei dem Ausführungsbeispiel der Figur 4 ist die Innenfläche 9 relativ zur Außenfläche 8 geneigt, der Neigungswinkel ist bei dem Ausführungsbeispiel der Figur 4 größer als bei dem Ausführungsbeispiel der Figur 5. Setzt man Ringe gemäß Figur 4 oder Figur 5 in eine Außenschale 1 ein, so wird ein im Ring 3 fixierter Gelenkeinsatz 4 relativ zur Außenschale 1 geneigt sein, der untere Rand des Gelenkeinsatzes 4 ragt dann auf einer Seite weiter von der Außenschale nach unten hervor als auf der gegenüberliegenden Seite. Eine solche Ausgestaltung ist in Figur 1 vor dem Zusammensetzen der Einzelteile der Hüftgelenkpfanne dargestellt.

Bei den Ausführungsbeispielen der Figuren 4 und 5 sind der obere Rand 13 und der untere Rand 14 des Rings 3 gegenüber einer Horizontalebene der Außenschale 1 ebenfalls geneigt. Bei dem Ausführungsbeispiel der Figur 6, das sonst weitgehend dem Ausführungsbeispiel der Figur 4 entspricht, ist jedoch der obere Rand 13 des Rings 3 parallel zu einer Horizontalebene der Außenschale 1 ausgebildet, der Ring 3 weist dementsprechend auf gegenüberliegenden Seiten eine unterschiedliche Bauhöhe auf.

Beim Ausführungsbeispiel der Figur 7 ist die Innenfläche 9 gegenüber der Außenfläche 8 seitlich versetzt, es ergibt sich somit eine exzentrische Anordnung der Innenfläche 9 gegenüber der Außenfläche 8. Wird ein solcher Ring in die Außenschale 1 eingesetzt, so wird auch die Gelenkfläche 10 exzentrisch in der Außenschale 1 positioniert.

Bei dem dargestellten Ausführungsbeispiel sind alle Ringe so ausgestaltet, daß sie zur Aufnahme ein- und desselben Gelenkeinsatzes 4 geeignet sind, das heißt die Form (Konizität) und die Abmessungen der Innenfläche 9 aller Ringe sind an die Abmessungen der Außenfläche 11 eines bestimmten Gelenkeinsatzes 4 angepaßt. Dadurch können die unterschiedlichen Positionierungen der Gelenkfläche 10 mit einem einzigen Gelenkeinsatz 4 erreicht werden. Die Gelenkfläche kann also in der beschriebenen Weise höher oder tiefer angeordnet, gegenüber der Außenschale 1 geneigt oder gegenüber der Außenschale 1 exzentrisch positioniert werden, bei geeigneter Ausgestaltung der Ringe 3 können auch die genannten unterschiedlichen Anordnungen und Orientierungen in beliebiger Weise kombiniert werden.

Der Ring 3 wird in der Gelenkpfanne in geeigneter Weise festgelegt. Dies ist in der Zeichnung nicht besonders dargestellt, hier können beliebige Festlegungsmittel verwendet werden, beispielsweise Einschraubgewinde oder Festlegung des Rings in der Außenschale durch einen auf die Außenschale 1 unten aufgesetzten Ring etc. Ebenso kann der Gelenkeinsatz 4 im Ring in beliebiger Weise festgelegt sein, auch hier sind alle Festlegungsmöglichkeiten eingeschlossen. Schließlich wird vorgesehen, daß sowohl der Ring 3 gegenüber der Außenschale 1 als auch der Gelenkeinsatz 4 gegenüber dem Ring 3 gegen eine Drehung gesichert sind, auch dies kann in beliebiger, an sich bekannter Weise erfolgen, beispielsweise durch das Eingreifen von Vorsprüngen in Ausnehmungen oder durch das Einsetzen von Drehsicherungen, die so, wohl in Ausnehmungen des einen Teils als auch in Ausnehmungen des anderen Teils eingreifen.

## Patentansprüche

1. Endoprothetische Hüftgelenkpfanne mit einer einen nach unten offenen Innenraum (2) aufweisenden Außenschale (1) und einem in diesen einsetzbaren Gelenkeinsatz (4) der eine kugelige Gelenkfläche (10) aufweist, wobei in den Innenraum (2) der Außenschale (1) ein Ring (3) eingesetzt ist, der in der Außenschale (1) fixiert ist und dessen Innenfläche (9) eine Anlagefläche für den Gelenkeinsatz (4) bildet, der an dem Ring (3) festgelegt ist, dadurch gekennzeichnet, daß die Innenfläche (9) des in die Außenschale (1) eingesetzten Rings (3) bei verschiedenen Ringen (3) relativ zu der Außenschale (1) unterschiedlich angeordnet ist.

2. Hüftgelenkpfanne nach Anspruch 1, dadurch gekennzeichnet, daß die Innenfläche (9) des in die Außenschale (1) eingesetzten Rings (3) bei verschiedenen Ringen (3) unterschiedlich tief in die Außenschale (1) eintaucht.

3. Hüftgelenkpfanne nach Anspruch 1 oder 2, dadurch gekennzeichnet, daß die Innenfläche (9) des in die Außenschale (1) eingesetzten Rings (3) bei verschiedenen Ringen (3) relativ zu der Außenschale (1) seitlich versetzt angeordnet ist.

4. Hüftgelenkpfanne nach einem der voranstehenden Ansprüche, dadurch gekennzeichnet, daß die Innenfläche (9) des in die Außenschale (1) eingesetzten Rings (3) bei verschiedenen Ringen (3) relativ zur Außenschale (1) unterschiedlich geneigt angeordnet ist.

5. Hüftgelenkpfanne nach einem der voranstehenden Ansprüche, dadurch gekennzeichnet, daß der untere Rand (14) eines Rings (3) längs seines Umfangs einen unterschiedlichen Abstand von einer Horizontalebene der Außenschale (1) aufweist.

6. Hüftgelenkpfanne nach einem der voranstehenden Ansprüche, dadurch gekennzeichnet, daß die Innenflächen (9) der Ringe (3) gleich geformt und dimensioniert sind.

7. Hüftgelenkpfanne nach einem der voranstehenden Ansprüche, dadurch gekennzeichnet, daß die Innenfläche (9) eines in die Außenschale (1) eingesetzten Rings (3) bei verschiedenen Ringen (3) relativ zu der Außenschale (1) unterschiedlich geformt und/oder dimensioniert ist.

8. Hüftgelenkpfanne nach einem der voranstehenden Ansprüche, dadurch gekennzeichnet, daß der Ring (3) eine konische Außenfläche (8) aufweist, die an einer konischen Innenfläche (5) des Innenraums (2) der Außenschale (1) anliegt.

9. Hüftgelenkpfanne nach einem der voranstehenden Ansprüche, dadurch gekennzeichnet, daß die Innenfläche (9) konisch ausgebildet ist und daß der Gelenkeinsatz (4) eine konische Außenfläche (11) trägt, die an der Innenfläche (9) anliegt.

10. Bausatz zum Aufbau einer Hüftgelenkpfanne mit einer Außenschale (1), einem Gelenkeinsatz (4) und einem zwischen Außenschale (1) und Gelenkeinsatz (4) angeordneten Ring (3), gekennzeichnet durch die Merkmale eines der Patentansprüche 1 bis 9.

## Claims

1. An endoprosthetic acetabular cup comprising an outer shell (1) having a downwardly open interior (2), and a joint insert (4) insertable into the latter and having a spherical joint surface (10), wherein a ring (3) is inserted into the interior (2) of the outer shell (1) and is fixed therein, its inner surface (9) forming a contact surface for the joint insert (4) fixed to the ring (3), characterised in that the inner surface (9) of the ring (3) inserted into the outer shell (1) is arranged differently relative to the outer shell (1) in different rings (3).

2. An acetabular cup according to claim 1, characterised in that the inner surface (9) of the ring (3) inserted into the outer shell (1) extends varyingly deeply into the outer shell (1) in different rings (3).

3. An acetabular cup according to claim 1 or 2, characterised in that the inner surface (9) of the ring (3) inserted into the outer shell (1) is arranged so as to be laterally offset relative to the outer shell (1) in various rings (3).

4. An acetabular cup according to any one of the preceding claims, characterised in that the inner surface (9) of the ring (3) inserted into the outer shell (1) is arranged at different angles relative to the outer shell (1) in different rings (3).

5. An acetabular cup according to any one of the preceding claims, characterised in that the distance of the lower edge (14) of a ring (3) from a horizontal plane of the outer shell (1) varies along its circumference.

6. An acetabular cup according to any one of the preceding claims, characterised in that the inner surfaces (9) of the rings (3) have the same shape and dimensions.

7. An acetabular cup according to any one of the preceding claims, characterised in that the inner surface (9) of a ring (3) inserted into the outer shell (1) is shaped and/or dimensioned differently relative to the outer shell (1) in different rings (3).

8. An acetabular cup according to any one of the preceding claims, characterised in that the ring (3) has a conical outer surface (8) which rests against a conical inner surface (5) of the interior (2) of the outer shell (1).

9. An acetabular cup according to any one of the preceding claims, characterised in that the inner surface (9) is conical and in that the joint insert (4) has a conical outer surface (11) resting against the inner surface (9).

10. A kit for constructing an acetabular cup comprising an outer shell (1), a joint insert (4) and a ring (3) arranged between the outer shell (1) and the joint insert (4), characterised by the features of any one of claims 1 to 9.

## Revendications

1. Capsule de hanche endoprothétique comportant une coquille externe (1) présentant une cavité interne (2) ouverte vers le bas et un insert d'articulation (4) insérable dans celle-ci qui comporte une surface d'articulation (10) sphérique, dans laquelle, dans la cavité interne (2) de la coquille externe (1) est insérée une bague (3) qui est fixée dans la coquille externe (1) et dont la surface interne (9) forme une surface d'appui pour l'insert d'articulation (4) qui est fixé sur la bague (3), caractérisée en ce que la surface interne (9) de la bague (3) insérée dans la coquille externe (1) est agencée de manière variable par rapport à la coquille externe (1) dans le cas de bagues (3) différentes.

2. Capsule de hanche selon la revendication 1, caractérisée en ce que la surface interne (9) de la bague (3) insérée dans la coquille externe (1) pénètre à une profondeur variable dans la coquille externe (1) dans le cas de bagues (3) différentes.

3. Capsule de hanche selon la revendication 1 ou 2, caractérisée en ce que la surface interne (9) de la bague (3) insérée dans la coquille externe (1) est agencée de manière décalée latéralement par rapport à la coquille externe (1) dans le cas de bagues (3) différentes.

4. Capsule de hanche selon l'une des revendications précédentes, caractérisée en ce que la surface interne (9) de la bague (3) insérée dans la coquille externe (1) est inclinée de manière variable par rapport à la coquille externe (1) dans le cas de bagues (3) différentes.

5. Capsule de hanche selon l'une des revendications précédentes, caractérisée en ce que le bord inférieur (14) d'une bague (3) présente le long de sa périphérie une distance variable avec un plan horizontal de la coquille externe (1).

6. Capsule de hanche selon l'une des revendications précédentes, caractérisée en ce que les surfaces internes (9) des bagues (3) sont formées et dimensionnées de la même manière.

7. Capsule de hanche selon l'une des revendications précédentes, caractérisée en ce que la surface interne (9) d'une bague (3) insérée dans la coquille externe (1) est formée et/ou dimensionnée de manière variable par rapport à la coquille externe (1) dans le cas de bagues (3) différentes.

8. Capsule de hanche selon l'une des revendications précédentes, caractérisée en ce que la bague (3) présente une surface externe (8) conique qui vient en appui sur une surface interne (5) conique de la cavité interne (2) de la coquille externe (1).

9. Capsule de hanche selon l'une des revendications précédentes, caractérisée en ce que la surface interne (9) est conique et en ce que l'insert d'articulation (4) porte une surface externe (11) conique qui vient en appui sur la surface interne (9).

10. Jeu de pièces pour la construction d'une capsule de hanche comportant une coquille externe (1), un insert d'articulation (4) et une bague (3) disposée entre la coquille externe (1) et l'insert d'articulation (4), caractérisé par les caractéristiques de l'une des revendications 1 à 9.
